# EUROPEAN PATENT APPLICATION

(11) **EP 4 582 522 A1**
(43) Date of publication of application: **09.07.2025**
(21) Application number: 24218434.9
(22) Date of filing: 09.12.2024
(51) Int. Cl.: C10L 3/08, C01B 3/50, F27D 17/20

(54) **HYDROGEN DELIVERY FOR CO2 MANAGEMENT IN CARBON-BASED MANUFACTURING**

(30) Priority: 02.01.2024 US 202418402522
(71) Applicant: Goodrich Corporation, Charlotte, NC 28217-4578 (US)
(72) Inventor: EMERSON, Sean C., Broad Brook, CT (US); CORDATOS, Haralambos, Colchester, CT (US); DARDAS, Zissis A., Worcester, MA (US); HALL, Daniel, Pueblo, CO (US); SHE, Ying, Rocky Hill, CT (US); SMITH, Kenneth D., East Longmeadow, MA (US); SUTTON, Leah Patrice, Pueblo, CT (US); YOUNG, Michael N., Colorado Springs, CO (US)
(74) Representative: Dehns

(57) **Abstract**

A system for carbon dioxide emission recovery is disclosed herein. The system includes a reformer (412, 512) configured to convert an input into hydrogen and carbon monoxide and a methanization reactor (410, 510) coupled to the reformer (412, 512). The methanization reactor (410, 510) is configured to receive carbon dioxide from one or more off gases of a carbon/carbon (C/C) preform production process, receive the hydrogen and the carbon monoxide from the reformer (412, 512), convert the hydrogen, the carbon monoxide, and the carbon dioxide via methanization to produce methane, and supply the produced methane to at least one of the C/C preform production process or another system for heat generation.

## Description

### FIELD

The present disclosure relates generally to carbon dioxide (CO₂) emission recovery, and more particularly, CO₂ emission recovery in carbon/carbon (C/C) preform production process.

### BACKGROUND

Composite bodies are utilized in various industries, including the aerospace industry. The composite bodies start with a preform that is formed using layers of textile material. Aircraft C/C brake manufacturing is one point source of carbon dioxide (CO₂) emissions. Other point sources of CO₂ emissions may include thermal protections systems and shaped composite bodies, among others. In such manufacturing, sustainability goals may be to reduce CO₂ emissions by a predetermined percentage by a future date. However, capturing CO₂ emissions is not only expensive but CO₂ emissions can also be problematic to store. This is especially true for CO₂ emission sources that do not generate enough CO₂ emissions to justify use of a pipeline for underground sequestration.

### SUMMARY

Disclosed herein is a system for carbon dioxide emission recovery. The system includes a reformer configured to convert an input into hydrogen and carbon monoxide and a methanization reactor coupled to the reformer. The methanization reactor is configured to receive carbon dioxide from one or more off gases of a carbon/carbon (C/C) preform production process, receive the hydrogen and the carbon monoxide from the reformer, convert the hydrogen, the carbon monoxide, and the carbon dioxide via methanization to produce methane, and supply the produced methane to at least one of the C/C preform production process or another system for heat generation.

In various embodiments, the system further includes a condenser coupled to the reformer and the methanization reactor. The condenser is configured to receive the hydrogen, the carbon monoxide, and water from the reformer, condense the water into a liquid form, and supply the hydrogen and the carbon monoxide to the methanization reactor. In various embodiments, the input includes a natural gas and steam. In various embodiments, the input includes a hydrocarbon and steam.

In various embodiments, the system further includes a separator coupled to the reformer. The separator is configured to receive one or more off gases from the C/C preform production process, separate one or more hydrocarbons from the one or more off gases, and supply the one or more hydrocarbons as the input to the reformer. In various embodiments, the system further includes a steam source coupled to the reformer and configured to provide a steam to the reformer and a natural gas input coupled to the reformer and configured to provide natural gas to the reformer. The input to the reformer includes the steam, the one or more hydrocarbons, and the natural gas are the input to the reformer.

In various embodiments, the system further includes a burner/steam generator coupled to the C/C preform production process and the reformer. The burner/steam generator is configured to receive the one or more off gases from the C/C preform production process, receive an air input, receive a water input, and output a steam to the reformer, wherein the input to the reformer includes the steam. In various embodiments, the system further includes a condenser coupled to the methanization reactor and the burner/steam generator, wherein the methanization reactor produces the methane and steam and wherein the condenser condenses the steam to water which is supplied to the burner/steam generator.

Also disclosed herein is a system for carbon dioxide emission recovery including a carbon/carbon (C/C) preform production component, a burner/steam generator coupled to the C/C preform production component and configured to receive one or more off gases from the C/C preform production component, receive an air input, and produce steam and carbon dioxide as an output, a reformer coupled to the burner/steam generator and configured to receive the steam from the burner/steam generator and a hydrocarbon from a hydrocarbon source and output hydrogen and carbon monoxide, a methanization reactor coupled to the burner/steam generator and the reformer, the methanization reactor configured to receive the hydrogen from the reformer and the carbon dioxide from the burner/steam generator and output methane and steam, wherein the methane is supplied back to the C/C preform production component.

In various embodiments, the system further includes a separator coupled to the C/C preform production component and the reformer, the separator configured to receive the one or more off gases and output the hydrogen and hydrocarbons to the reformer, wherein the separator is the hydrocarbon source. In various embodiments, the system further includes a natural gas source coupled to the reformer, wherein the natural gas source and the separator are the hydrocarbon source.

In various embodiments, the hydrocarbon source is a natural gas source. In various embodiments, a condenser coupled to the reformer and the methanization reactor. The condenser is configured to receive the hydrogen, the carbon monoxide, and water from the reformer, condense the water into a liquid form to be supplied to the burner/steam generator, and supply the hydrogen and the carbon monoxide to the methanization reactor. In various embodiments, a condenser coupled to the methanization reactor and the burner/steam generator, wherein the methanization reactor produces the methane and steam and wherein the condenser condenses the steam to water which is supplied to the burner/steam generator.

In various embodiments, the system further includes a separator coupled to the burner/steam generator and the methanization reactor and configured to receive a byproduct from the burner/steam generator, separate the carbon dioxide from the by product, and supply the carbon dioxide to the methanization reactor.

Also disclosed herein is a method for carbon dioxide emission recovery. The method includes receiving, by a methanization reactor, carbon dioxide separated from one or more off gases of a carbon/carbon (C/C) preform production component, receiving, by the methanization reactor, a hydrogen supply from a reformer, the hydrogen supply being reformed from a hydrocarbon source, converting, by the methanization reactor, the carbon dioxide and the hydrogen supply via methanization to produce methane, and supplying, by the methanization reactor, the produced methane to the C/C preform production component.

In various embodiments, the hydrocarbon source is a natural gas source. In various embodiments, the hydrocarbon source is a separator, the separator separating one or more hydrocarbons from the off gases of the C/C preform production component. In various embodiments, the hydrocarbon source includes a natural gas source and a separator, wherein the separator separates the one or more hydrocarbons from the off gases of the C/C preform production component. In various embodiments, the method further includes output, by the methanization reactor, steam to a condenser to be converted into liquid water for use by a system for steam generation.

The foregoing features and elements may be combined in any combination, without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the following detailed description and claims in connection with the following drawings. While the drawings illustrate various embodiments employing the principles described herein, the drawings do not limit the scope of the claims.
FIG. 1A illustrates an exemplary aircraft having a brake system, in accordance with various embodiments.
FIG. 1B illustrates a cross-sectional view of a brake assembly, in accordance with various embodiments.
FIG. 2 illustrates a fibrous preform, in accordance with various embodiments.
FIG. 3 illustrates a fibrous preform in a carbonization furnace, in accordance with various embodiments.
FIG. 4 illustrates a system for CO2 emission recovery by converting recovered CO2 into other products, in accordance with various embodiments.
FIG. 5 illustrates a system for CO2 emission recovery by converting recovered CO2 into other products, in accordance with various embodiments.
FIG. 6 illustrates a system for CO2 emission recovery by converting recovered CO2 into other products, in accordance with various embodiments.
FIG. 7 illustrates a method for CO2 emission recovery by converting recovered CO2 into other products, in accordance with various embodiments.

### DETAILED DESCRIPTION

The following detailed description of various embodiments herein makes reference to the accompanying drawings, which show various embodiments by way of illustration. While these various embodiments are described in sufficient detail to enable those skilled in the art to practice the disclosure, it should be understood that other embodiments may be realized and that changes may be made without departing from the scope of the disclosure. Thus, the detailed description herein is presented for purposes of illustration only and not of limitation. While these exemplary embodiments are described in sufficient detail to enable those skilled in the art to practice the invention, it should be understood that other embodiments may be realized and that logical, chemical and mechanical changes may be made without departing from the spirit and scope of the invention. For example, the steps recited in any of the method or process descriptions may be executed in any order and are not necessarily limited to the order presented. Furthermore, any reference to singular includes plural embodiments, and any reference to more than one component or step may include a singular embodiment or step. Also, any reference to attached, fixed, connected, or the like may include permanent, removable, temporary, partial, full or any other possible attachment option. Additionally, any reference to without contact (or similar phrases) may also include reduced contact or minimal contact. It should also be understood that unless specifically stated otherwise, references to "a," "an" or "the" may include one or more than one and that reference to an item in the singular may also include the item in the plural. Further, all ranges may include upper and lower values and all ranges and ratio limits disclosed herein may be combined.

In typical carbon/carbon (C/C) preform production process, such as aircraft C/C brake manufacturing, thermal protections systems, and shaped composite bodies, among others, carbon dioxide (CO2) emissions account for a substantial percentage of a total CO2 emissions for some corporations. In such corporations, sustainability goals may be to decarbonize operations, e.g., by reducing CO2 emissions, by a predetermined percentage by a future date. While technology for capturing CO2 emissions may be well-established and already in practice for applications ranging from enhanced oil recovery to carbonation of soft drinks, one of the costs associated with capturing CO2 emissions is the logistics and additional energy input associated with CO2 emission sequestration, which, for large point sources such as power plants, involves pressurization and transfer via pipeline to underground storage locations. Such CO2 emission recovery is unlikely to become a viable solution for relatively small CO2 emission point sources.

Disclosed herein are systems and methods for CO2 emission recovery by converting the recovered CO2 into other products. Accordingly, in various embodiments, in manufacturing processes where other gases such as hydrogen (H₂) are also generated as a byproduct, the H₂ may react catalytically with the recovered CO₂ to form methane (CH₄) via a methanization reaction, i.e., the main species in natural gas:

CO₂ + 4H₂ → CH₄ + 2H₂O

This methanization reaction may also be referred to as a Sabatier reaction. In manufacturing processes, such as preform forming manufacturing, the methanization reaction offers the most straightforward path for management of CO₂ emissions, given that the preform forming manufacturing process already typically utilizes methane (natural gas) for operation and given that the C/C brake manufacturing process generates excess H₂. While the excess H₂ generated by the C/C brake manufacturing process may not be enough to account for the entire CO₂ emissions, the H₂ may be supplemented with additional "green hydrogen" (i.e., produced sustainably), which is projected to become widely available. Additionally, the water (H₂O) produced from the methanization reactor may also be recycled back into a steam generator used for the C/C brake manufacturing process.

The systems and methods disclosed herein further include, in various embodiments, a reformer to produce H₂ for the Sabatier reactor. In various embodiments, the off gases from the C/C brake manufacturing process may be fed into the reformer. In various embodiments, the reformer eliminates the use of a separator to feed the methanization reactor as the output of the reformer may be fed directly into the methanization reactor. The reformer, in various embodiments, reforms natural gas available in the system, as well as hydrocarbons present in the off gases, into syngas which comprises hydrogen (H₂) and carbon monoxide (CO):

CH₄ + H₂O → CO + 3H₂

In various embodiments, the CO produced by the reformer may undergo hydrogenation to produce additional methane in the methanization reactor:

CO + 3H₂ → CH₄ + H₂O

This process differs, in various embodiments, from traditional systems that perform the reforming process after the methanization reactor separates the H₂ from the CO.

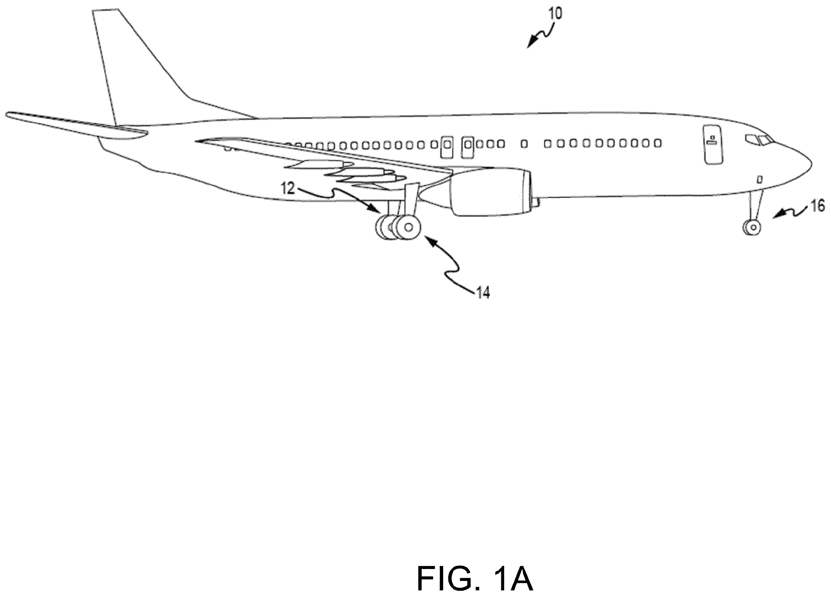

Referring now to FIG. 1A, an aircraft 10 is illustrated, in accordance with various embodiments. Aircraft 10 includes landing gear, which may include a left main landing gear 12, a right main landing gear 14, and a nose landing gear 16. The landing gear support aircraft 10 when it is not flying, allowing aircraft 10 to taxi, take off, and land without damage. While the disclosure refers to the three landing gear configurations just described, the disclosure nevertheless contemplates any number of landing gear configurations.

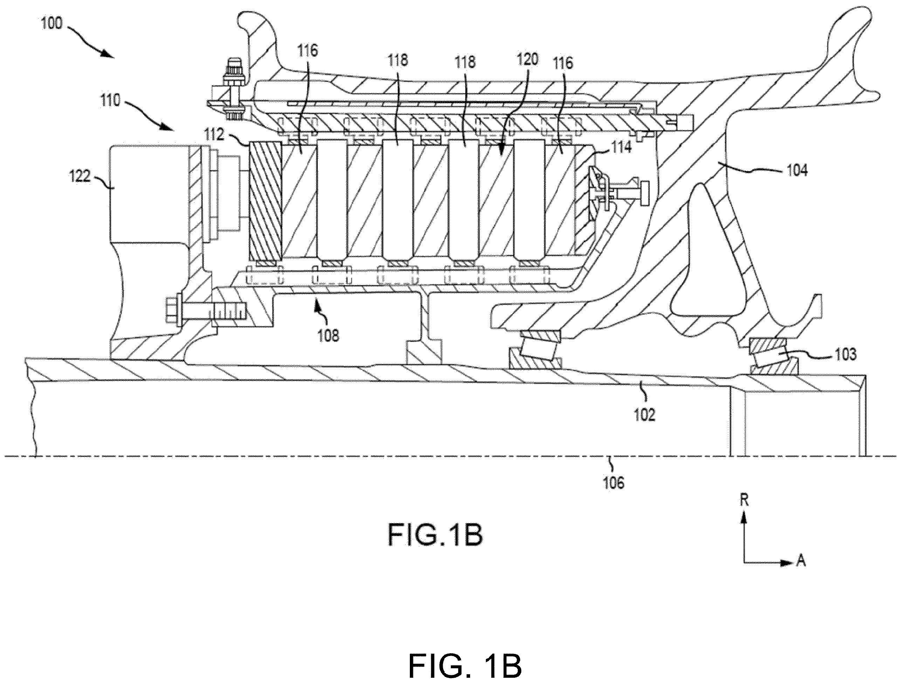

Referring now to FIG. 1B, a schematically depicted a brake mechanism 100 is illustrated configured for use on a landing gear, in accordance with various embodiments. For example, each of left main landing gear 12 and right main landing gear 14 described above with reference to FIG. 1A. In various embodiments, brake mechanism 100 is mounted on an axle 102 for use with a wheel 104 disposed on and configured to rotate about axle 102 via one or more bearing assemblies 103. A central axis 106 extends through axle 102 and defines a center of rotation of wheel 104. As used herein, the term "radial" refers to directions perpendicular to a central axis 106 of fibrous preform, the term "axial" refers to direction parallel to central axis 106, and the term "circumferential" reference to directions about central axis 106. A torque plate barrel 108 (sometimes referred to as a torque tube or a torque plate) is aligned concentrically with central axis 106, and wheel 104 is rotatable relative to torque plate barrel 108.

Brake mechanism 100 includes a piston assembly 110, a pressure plate 112 disposed adjacent piston assembly 110, an end plate 114 positioned a distal location from piston assembly 110, and a plurality of rotor disks 116 interleaved with a plurality of stator disks 118 positioned intermediate pressure plate 112 and end plate 114. Pressure plate 112, the plurality of rotor disks 116, the plurality of stator disks 118, and end plate 114 together form a brake heat sink or brake stack 120. Pressure plate 112, end plate 114, and the plurality of stator disks 118 are mounted to torque plate barrel 108 and remain rotationally stationary relative to axle 102. The plurality of rotor disks 116 is mounted to wheel 104 and rotate with respect to each of pressure plate 112, end plate 114, and the plurality of stator disks 118.

An actuating mechanism for brake mechanism 100 includes a plurality of piston assemblies, including piston assembly 110, circumferentially spaced around a piston housing 122 (only one piston assembly is illustrated in FIG. 1B). Upon actuation, the plurality of piston assemblies affects a braking action by urging pressure plate 112 and the plurality of stator disks 118 into frictional engagement with the plurality of rotor disks 116 and against end plate 114. Through compression of the plurality of rotor disks 116 and the plurality of stator disks 118 between pressure plate 112 and end plate 114, the resulting frictional contact slows or stops or otherwise prevents rotation of wheel 104. In various embodiments, the brake disks of brake mechanism 100 (e.g., rotor disks 116 and stator disks 118) are fabricated from various composite materials, such as, for example, carbon/carbon (C/C) composite or ceramic matrix composite (CMCs), that enable the brake disks to withstand and dissipate the heat generated during and following a braking action.

In accordance with various embodiments, rotor disks 116 and/or stator disks 118 are each comprised of a carbon-carbon (C/C) material having a high specific heat particulate interspersed throughout the rotor disks 116 and/or stator disks 118, where high specific heat particulate includes any particulate or powder (typically ceramic) that raises the specific heat of the disk above that of C/C composite alone. For example, in various embodiments, rotor disks 116 and/or stator disks 118 may comprise a C/C composite that includes a percentage of boron, a boron component, or other material having a high specific heat (i.e., a specific heat greater than the specific heat of the C/C composite alone). In various embodiments, rotor disks 116 and/or stator disks 118 may comprise a C/C composite with a percentage of boron carbide (B₄C) disposed substantially throughout the disk.

In various embodiments, the process of interspersing the boron carbide (or other high specific heat component) into the C/C composite is performed by a slurry infiltration process. As described in further detail below, the slurry may infiltrate a fiber preform employed to form the C/C composite in the radial, or in-plane, direction. The slurry may infiltrate via through thickness infiltration of the carbonize preforms. In various embodiments infiltrating in the radial, as opposed to the axial, direction may allow greater volumes and/or larger size particles to be infiltrated. Increasing the volume and/or size of the particles may facilitate the densification process by increasing the surface area available for the matrix material to bond to and by decreasing the open, or empty, volume within the preform. In-plane infiltration may also allow thicker fibrous preforms (e.g., fibrous preforms including a greater number of fiber layers) to be used in the manufacture of rotor disks 116 and/or stator disks 118, as the compared to axial infiltration.

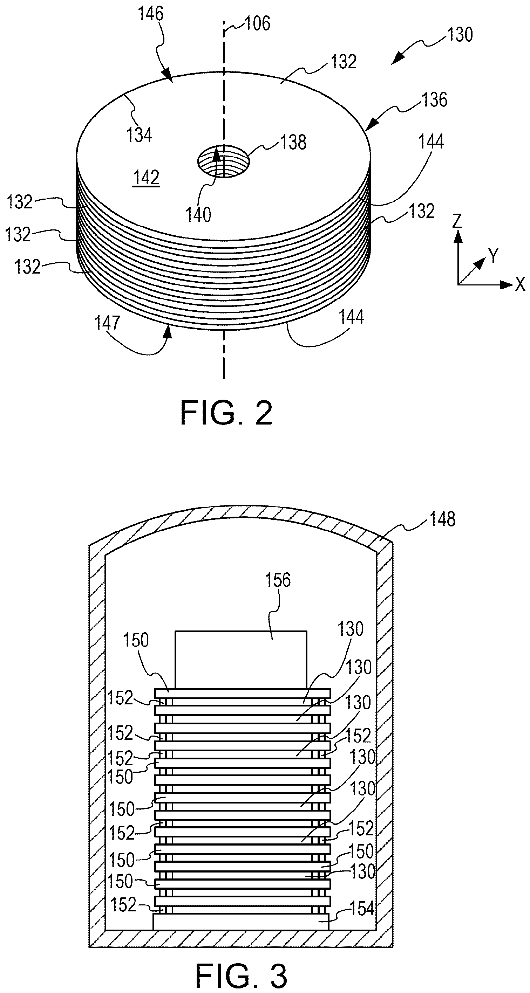

Referring now to FIG. 2, a fibrous preform 130 is illustrated, in accordance with various embodiments. Fibrous preform 130 may be employed to form a rotor disk 116 or a stator disk 118, as described above. Fibrous preform 130 may comprise a porous structure comprised of a plurality of stacked textile layers 132. Each textile layer 132 having a thickness in a first dimension (i.e., the Z-direction) that may be substantially less than a thickness of the layer 132 in the other two dimensions (i.e., the X-direction and the Y-direction). As used herein, the "in-plane" direction refers to directions parallel to the thicker two dimensions (i.e., parallel to the X and Y directions and perpendicular to the Z-direction).

A porous structure may comprise any structure derived from a fibrous material such as carbon fibers or the like. In various embodiments, the carbon fibers may be derived from polyacrylonitrile (PAN), rayon (synthetic fiber derived from cellulose), oxidized polyacrylonitrile fiber (OPF), pitch, or the like. The starting fiber may be pre-oxidized PAN or fully carbonized commercial carbon fiber. Fibrous preform 130 may be prepared by needling the textile layers 132 of fibrous preform 130. Needling the textile layers 132 of fibrous preform 130 tends to push fibers from one layer 132 to the next layer 132, thereby forming z-fibers that extend axially across the layers 132. Needling pulls fibers from the in-plane direction and forces the fibers into the z-fiber direction. After needling, fibrous preform 130 may comprise fibers extending in three different directions: the radial direction, the circumferential direction, and the axial direction (or the X, Y, and Z directions).

Fibrous preform 130 may be fabricated using a net shape preforming technology or may be cut from a needled board. Fibrous preform 130 may be a lay-up of woven, braided or knitted textile layers 132. The fibrous material may be in the form of chopped carbon fibers molded to form layers 132. Prior to the densification process, the fibrous material may be formed into a preform having any desired shape or form. For example, the fibrous preform may be in the form of a disk having any shape such as, for example, a polygon, a cylinder, a triangle, annular, square, rectangle, pentagon, hexagon, octagon, or the like. In various embodiments, layers 132 and fibrous preform 130 may have a generally annular shape. In accordance with various embodiments, the outer circumferential (or outer perimeter) surfaces 134 of layers 132 may form an outer diameter (OD) 136 of fibrous preform 130, and the inner circumferential (or inner perimeter) surfaces 138 of layers 132 may form an inner diameter (ID) 140 of fibrous preform 130. Each layer 132 includes a first axial face 142 and a second axial face 144 opposite the first axial face 142. First and second axial faces 142, 144 extend from outer circumferential surface 134 to inner circumferential surface 138. Layers 132 are stacked such that the first axial face 142 of one layer 132 is oriented toward the second axial face 144 of the adjacent layer 132. First axial face 142 of the uppermost layer 132 forms the upper axial end 146 of fibrous preform 130 and the second axial face 144 of the bottommost layer 132 forms the lower axial end 147 of fibrous preform 130 (i.e., the two layers 132 that are farther apart from one another in the axial direction form the axial ends 146, 147 of the fibrous preform).

As shown in FIG. 3, in accordance with various embodiments, fibrous preforms 130 being placed in a furnace 148 for carbonization is illustrated. The carbonization process may be employed to convert the fibers of fibrous preforms 130 into pure carbon fibers, as used herein only "pure carbon fibers" means carbon fibers comprised of at least 99% carbon. The carbonization process is distinguished from the densification process described below in that the densification process involves infiltrating the pores of fibrous preform 130 and depositing a matrix (e.g., carbon, phenolic resin, or any other desired matrix material) within and around the carbon fibers of the fibrous preform, and the carbonization process refers to the process of converting the fibers of fibrous preform 130 into pure carbon fibers.

In various embodiments, a plurality of fibrous preforms 130 may be placed on top of one another with separator plates 150 and spacing stops 152 disposed between adjacent fibrous preforms 130. For example, the bottommost fibrous preform 130 may be placed on a base plate 154 at the bottom of carbonization furnace 148. A separator plate 150 may be placed on top of the bottommost fibrous preform 130. Another fibrous preform 130 may then be placed on the separator plate 150, and another separator plate 150 may be placed on that fibrous preform 130. A stack of fibrous preforms 130 and separator plates 150 may be constructed in this manner, with each fibrous preform 130 being separated from superjacent and subjacent fibrous preforms 130 by separator plates 150. Stops 152 may be placed between each of the separator plates 150. Stops 152 may comprise a height that is less than the thickness of fibrous preform 130 prior to carbonization. Stops 152 may define a target thickness of fibrous preform 130 after carbonization. In that regard, after the stack of fibrous preforms 130 is constructed, and before the carbonization process has started, gaps may exist between stops 152 and adjacent separator plates 150. During carbonization, a compressive load may be applied to fibrous preforms 130, thereby compressing fibrous preforms 130 until stops 152 contact adjacent separator plates 150.

In various embodiments, compressive pressure may be applied to fibrous preforms 130 during the carbonization. The compressive pressure may be applied by placing a weight 156 over fibrous preforms 130, or by applying a compressive load to fibrous preforms 130 by other suitable means. The compressive pressure may be applied along the direction of the z-fibers. It will be appreciated by those skilled in the art that the mass of weight 156 and/or the compressive force generated by weight 156 may vary depending on the size of fibrous preforms 130, the pre-carbonization fiber volume of fibrous preforms 130, the desired post-carbonization fiber volume of fibrous preforms 130, and/or the number fibrous preforms 130 being compressed. As used herein, "fiber volume" refers the percentage of the total volume of the fibrous preform that is formed by the fibers of the fibrous preform. For example, a fiber volume of 18% means the fibers of the fibrous preform form 18% of the total volume of fibrous preform. In various embodiments, after carbonization, fibrous preform 130 includes a fiber volume of between 10% and 50%. In various embodiments, after carbonization, fibrous preform 130 includes a fiber volume of between 15% and 25%. In various embodiments, fibrous preforms 130 having a low fiber volume may be desirable for the infiltration methods disclosed herein. In various embodiments, after carbonization, fibrous preform 130 may comprise a fiber volume of less than 25%. For example, in various embodiments, after carbonization, fibrous preform 130 may comprise a fiber volume of 21% or, in various embodiments, fibrous preform 130 may comprise a fiber volume of 18%.

After carbonization, fibrous preform 130, may be densified using, for example, CVI. In various embodiments, prior to densification, fibrous preform 130 is infiltrated with a slurry including a high specific heat particulate. For example, in various embodiments, fibrous preform 130 is infiltrated with a ceramic slurry (i.e., a slurry comprised of a liquid carrier and ceramic particulates). In various embodiments, the slurry infiltration process includes preparation of a slurry including a ceramic particulate (e.g., an aqueous B₄C-based slurry) and immersing the carbonized fibrous preform 130 into the slurry for a period of time sufficient for the particulate (e.g., the B₄C) to infiltrate fibrous preform 130.

The slurry includes sacrificial fibers. For instance, the sacrificial fibers / particulates are mixed into the slurry. The sacrificial fibers may be a polyethylene or polyester fiber such that, in response to heating to carbon CVI temperatures (e.g., about 100° F to about 205° F (about 38° C to about 96° C)) will burn. In various embodiments, the preform is heated up to about 1000° C in order to cause the sacrificial fibers for burn away, or decompose. When the sacrificial fibers are burned away, or decompose, channels are formed, thus improving carbon CVI infiltration. For instance, the channels provide a pathway for the CVI to get to in. However, polyester or polyethylene fibers may be too large to incorporate directly into the B₄C slurry. Accordingly, the sacrificial fibers may be injected into fibrous preform 130. For instance, the sacrificial fibers may be injected using a syringe or other means, prior to completing B₄C slurry infiltration. By injecting using a syringe, the sacrificial fibers may be distributed in the matrix of the boron carbide.

Slurry may include high specific heat particulate and a liquid carrier (such as, for example, water and/or alcohol). In various embodiments, slurry may further include a high specific heat particulate, a binder (e.g., a polymeric adhesive or polyvinyl acetate), and a liquid carrier (e.g., water). It will be appreciated by those skilled in the art that liquid carriers other than water may be used and that the type and/or volume of liquid carrier and/or of binder may be selected based on the composition of the high specific heat particulate. In various embodiments, slurry may be a B₄C-based slurry and may be prepared by mixing B₄C powder in water with appropriate additives, such as wetting agents and dispersants. The B₄C powder may comprise particulates having an average particle size from sub-micron up to about 30 microns. As used in the previous context only, "about" means ± 5 microns.

The slurry further includes the sacrificial fibers. The sacrificial fibers are added to the solution of the slurry. In various embodiments, the sacrificial fibers make up about 5% of the slurry solution. In various embodiments, the sacrificial fibers make up about 10 % of the slurry solution. For instance, the sacrificial fibers make up about 0.5% to about 10% of the slurry. The sacrificial fibers may be a micro polyester particle. The slurry solution may be loaded into the syringe. A user may then use the syringe to inject the slurry, including the sacrificial fibers, into fibrous preform 130. The user may inject fibrous preform 130 in a plurality of locations. For instance, the user may inject/poke fibrous preform 130 at ten locations. In various embodiments, a bank of syringes may be provided to operate as an assembly-line system to prepare multiple fibrous preforms may be injected simultaneously with the sacrificial fibers.

Infiltration using a syringe facilitates achieving a uniform distribution of the sacrificial fibers, which in turn improves carbon CVI infiltration once the fibers are burned around during the heat-up to carbon CVI process temperature. By raising the thermal capacity of the material, more energy may be absorbed per unit mass. Accordingly, the brakes may be manufactured smaller, for instance. Further, the friction and wear characteristics may be improved.

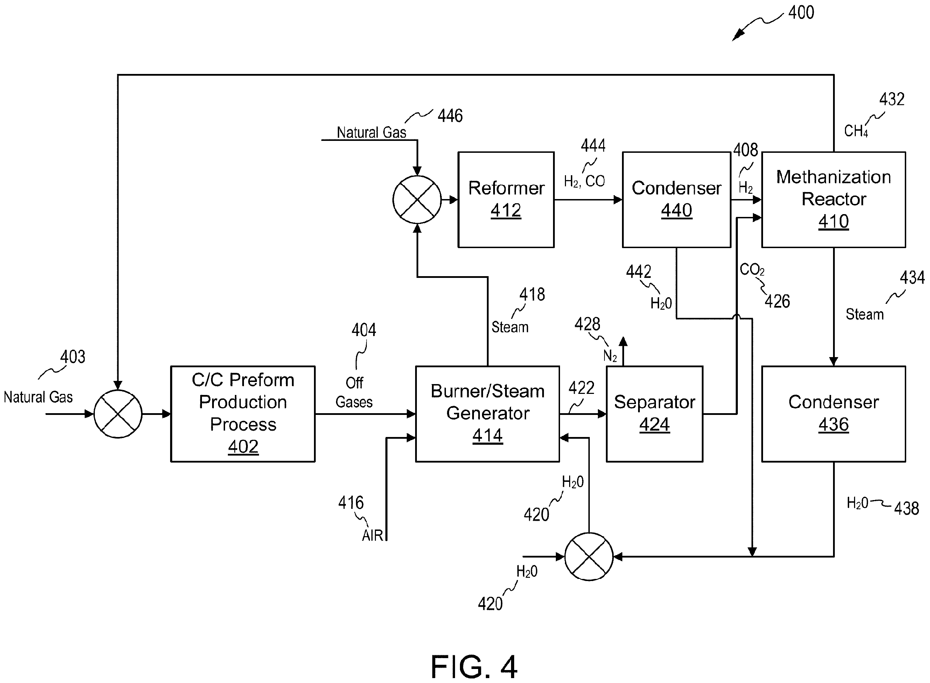

Referring now to FIG. 4, a system 400 for CO₂ emission recovery by converting recovered CO₂ into other products is illustrated, in accordance with various embodiments. As described previously, the process illustrated and described with respect to FIG. 3 includes the emission of off gases that include but are not limited to hydrogen (H₂) and carbon dioxide (CO₂). System 400 includes a C/C preform production process 402, a methanization reactor 410, a reformer 412, a condenser 440, a burner/steam generator 414, a separator 424, and a condenser 436.

In various embodiments, C/C preform production process 402 utilizes natural gas 403 to perform various operations in the production of the aircraft brakes. In various embodiments, C/C preform production process 402 includes components, machines, and systems for producing C/C preforms. In various embodiments, C/C preform production process 402 may be a chemical vapor infiltration (CVI) process. In various embodiments, C/C preform production process 402 may be a chemical vapor deposition (CVD) process. However, in various embodiments, during C/C preform production process 402, various off gases 404, i.e., hydrocarbons, are produced. Accordingly, in system 400, C/C preform production process 402 feeds the various off gases 404, i.e., hydrocarbons, CO2, H2, and nitrogen (N2), among others, into burner/steam generator 414. Burner/steam generator 414 combines the various off gases 404, incoming air 416, and incoming water (H₂O) 420. Burner/steam generator 414 burns the combined off gases 404, air 416, and H₂O 420 to generate steam 418. In various embodiments, burner/steam generator 404 may be a boiler. Steam 418 and incoming natural gas 446, including methane (CH₄), are mixed and fed into a reformer 412. Reformer 412 reforms the mixture of steam 418 and natural gas 446 into a syngas 444 containing hydrogen (H₂) and carbon monoxide (CO) according to the equation CH₄ + H₂O → CO + 3H₂. Syngas 444 is fed into a condenser 440 that produces H₂O 442, that is fed back into burner/steam generator 414, and H₂ 408 that is fed into methanization reactor 410. In various embodiments, steam 418 may be utilized to operate other components of C/C preform production process 402.

During the operations performed by burner/steam generator 414, in addition to generating steam 418, burner/steam generator 414 generates byproducts 422, such as such as CO₂, H₂O, and N₂, among others, which burner/steam generator 414 feeds into separator 424. Separator 424 may operate to separate CO₂ 426 from byproducts 422, which may be fed into methanization reactor 410, to separate N₂ 428 from byproducts 422, which may be released into the atmosphere, and to separate H₂O 430 from byproducts 422, which may be fed back into burner/steam generator 414 along with H₂O 420. Separator 424 may include one or more of absorption, adsorption, cryogenic distillation, captured by solid sorbents or solvents such as mono-ethanol amine (MEA), or membrane separation, among others for the separation process.

In various embodiments, methanization reactor 410 utilizes the incoming H₂ 408 and CO₂ 426 to perform a methanization process in order to produce methane (CH₄) 432, which is fed into C/C preform production process 402, or another system, to perform various operation in the production the aircraft brakes, such as heat generation. In various embodiments, during the production of CH₄ 432, methanization reactor 410 also produces steam 434, which is fed into condenser 436. In various embodiments, the condenser 436 condenses the steam 434 to generate H₂O 438, which may be fed into burner/steam generator 414 along with H₂O 420 and H₂O 430. Accordingly, system 400 provides for CO₂ emission recovery by converting the recovered CO₂ into other products that may be utilized by C/C preform production process 402.

In various embodiments, condenser 436 may include a first output for H₂0 438, as illustrated, and a second output that feeds into reformer 412. The first output feeds back into burner/steam generator 414 as previously described. The second output may feed a combination of steam and CH₄ to reformer 412. More specifically, the combination of steam and CH₄ may be combined with steam 418 and natural gas 446 before being fed into reformer 412. With this configuration system 400 is able to extract additional hydrocarbons for use in methanization reactor 410.

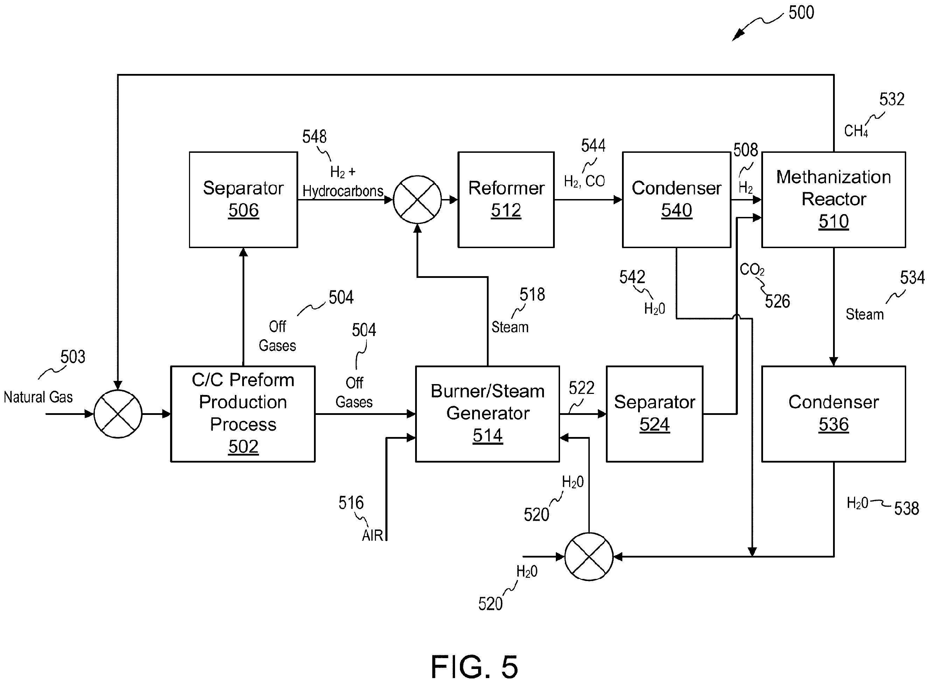

Referring now to FIG. 5, a system 500 for CO₂ emission recovery by converting recovered CO₂ into other products is illustrated, in accordance with various embodiments. As described previously, the process illustrated and described with respect to FIG. 3 includes the emission of off gases that include but are not limited to hydrogen (H₂) and carbon dioxide (CO₂). System 500 includes similar components to system 400 including a C/C preform production process 502, a methanization reactor 510, a reformer 512, a condenser 540, a burner/steam generator 514, a separator 524, and a condenser 536, descriptions of which may not be repeated below. System 500 further includes a separator 506.

In various embodiments, C/C preform production process 502 utilizes natural gas 503 to perform various operations in the production of the aircraft brakes. However, in various embodiments, during C/C preform production process 502, various off gases 504, i.e., hydrocarbons, are produced. Accordingly, in system 500, C/C preform production process 502 feeds the various off gases 504, i.e., hydrocarbons, CO₂, H₂, and nitrogen (N₂), among others, into separator 506 and into burner/steam generator 514. In various embodiments, separator 506 separates H₂ and various hydrocarbons from the various off gases 504 using one or more of membrane separation, pressure swing absorption (PSA), captured by solid sorbents or solvents such as mono-ethanol amine (MEA), or cryogenic distillation, among others. In various embodiments, separator 506 feeds output 548 into reformer 512 along with steam 518 generated by burner/steam generator 514.

Burner/steam generator 514 combines the various off gases 504, incoming air 516, and incoming water (H₂O) 520. Burner/steam generator 514 burns the combined off gases 504 in air 516to heat up H₂O 520 to generate steam 518. Steam 518 and output 548 are mixed and fed into reformer 512. Reformer 512 reforms the mixture of steam 518 and output 548 into a syngas 544 containing hydrogen (H₂) and carbon monoxide (CO) according to the equation CH₄ + H₂O - CO + 3H₂. Syngas 544 is fed into a condenser 540 that produces H₂O 542, that is fed back into burner/steam generator 514, and H₂ 508 that is fed into methanization reactor 510. In various embodiments, steam 518 may be utilized to operate other components of C/C preform production process 502.

During the operations performed by burner/steam generator 514, in addition to generating steam 518, burner/steam generator 514 generates byproducts 522, such as such as CO₂, H₂O, and N₂, among others, which burner/steam generator 514 feeds into separator 524. Separator 524 may operate to separate CO₂ 526 from byproducts 522, which may be fed into methanization reactor 510, to separate N₂ 528 from byproducts 522, which may be released into the atmosphere, and to separate H₂O 530 from byproducts 522, which may be fed back into burner/steam generator 514 along with H₂O 520. Separator 524 may include one or more of absorption, adsorption, cryogenic distillation, captured by solid sorbents or solvents such as mono-ethanol amine (MEA), or membrane separation, among others for the separation process.

In various embodiments, methanization reactor 510 utilizes the incoming H₂ 508 and CO₂ 526 to perform a methanization process in order to produce methane (CH₄) 532, which is fed into C/C preform production process 502, or another system, to perform various operation in the production the aircraft brakes, such as heat generation. In various embodiments, during the production of CH₄ 532, methanization reactor 510 also produces steam 534, which is fed into condenser 536. In various embodiments, the condenser 536 condenses the steam 534 to generate H₂O 538, which may be fed into burner/steam generator 514 along with H₂O 520 and H₂O 530. In various embodiments, as described above in FIG. 4, condenser 536 may include a second output that feeds steam, including CH₄, back to reformer 412. Accordingly, system 500 provides for CO₂ emission recovery by converting the recovered CO₂ into other products that may be utilized by C/C preform production process 502.

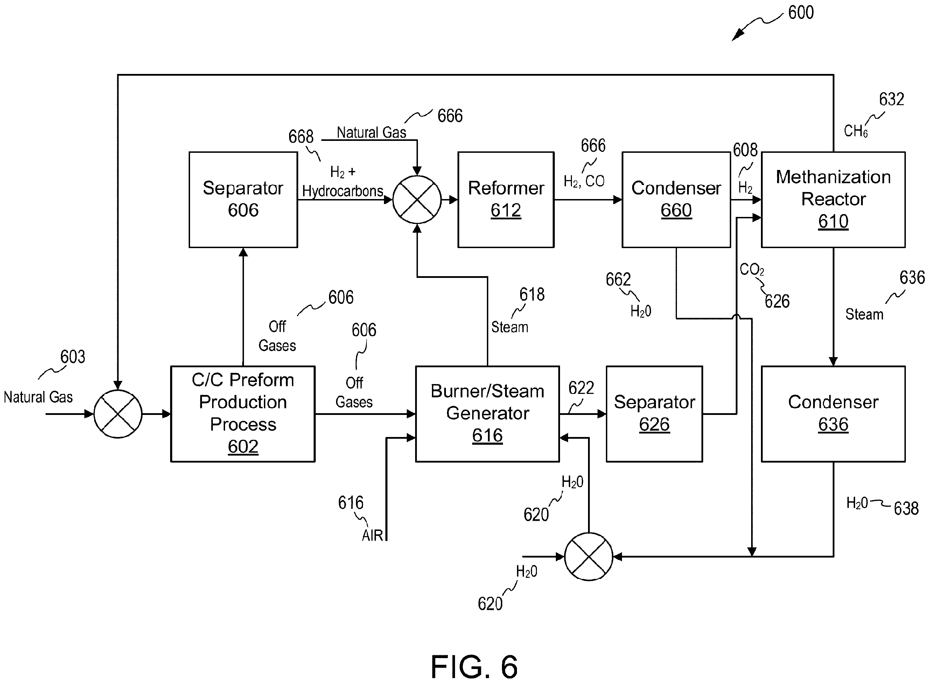

Referring now to FIG. 6, a system 600 for CO₂ emission recovery by converting recovered CO₂ into other products is illustrated, in accordance with various embodiments. As described previously, the process illustrated and described with respect to FIG. 3 includes the emission of off gases that include but are not limited to hydrogen (H₂) and carbon dioxide (CO₂). System 600 includes similar components to system 400 and system 500 including a C/C preform production process 602, a separator 606, a methanization reactor 610, a reformer 612, a condenser 640, a burner/steam generator 614, a separator 624, and a condenser 636, and a natural gas 446 input, descriptions of which are not be repeated below. System 600 is a combination of system 400 and system 500 and combines steam 618, output 668 from separator 606, and natural gas 666 as inputs into reformer 612.

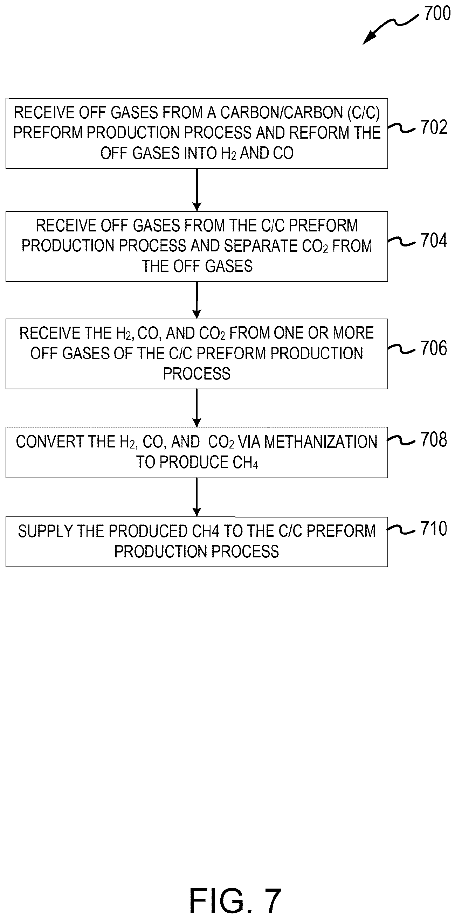

Referring now to FIG. 7, a method 700 for CO₂ emission recovery by converting recovered CO₂ into other products is illustrated, in accordance with various embodiments. For ease of description, method 700 is described with reference to FIGS. 4, 5, and 6. At block 702, a reformer receives one or more off gases from a carbon/carbon (C/C) preform production process and separates the off gases into H₂ and CO. At block 704, a separator receives one or more off gases from the C/C preform production process and separates CO₂ from the one or more off gases. At block 706, a methanization reactor receives the H₂, CO, and CO₂ reformed and separated from the one or more off gases of the C/C preform production process. At block 708, the methanization reactor converts the H₂, CO, and CO₂ via methanization to produce CH₄. At block 710, the methanization reactor supplies the produced CH₄ to the C/C preform production process.

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, Band C may be present in a single embodiment; for example, A and B, A and C, B and C, or A and B and C. Different cross-hatching is used throughout the figures to denote different parts but not necessarily to denote the same or different materials.

Systems, methods, and apparatus are provided herein. In the detailed description herein, references to "one embodiment," "an embodiment," "various embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

Numbers, percentages, or other values stated herein are intended to include that value, and also other values that are about or approximately equal to the stated value, as would be appreciated by one of ordinary skill in the art encompassed by various embodiments of the present disclosure. A stated value should therefore be interpreted broadly enough to encompass values that are at least close enough to the stated value to perform a desired function or achieve a desired result. The stated values include at least the variation to be expected in a suitable industrial process, and may include values that are within 5% of a stated value. Additionally, the terms "substantially," "about" or "approximately" as used herein represent an amount close to the stated amount that still performs a desired function or achieves a desired result. For example, the term "substantially," "about" or "approximately" may refer to an amount that is within 5% of a stated amount or value.

Furthermore, no element, component, or method step in the present disclosure is intended to be dedicated to the public regardless of whether the element, component, or method step is explicitly recited in the claims. As used herein, the terms "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus.

Finally, it should be understood that any of the above-described concepts can be used alone or in combination with any or all of the other above-described concepts. Although various embodiments have been disclosed and described, one of ordinary skill in this art would recognize that certain modifications would come within the scope of this disclosure. Accordingly, the description is not intended to be exhaustive or to limit the principles described or illustrated herein to any precise form. Many modifications and variations are possible in light of the above teaching.

## Claims

1. A system for carbon dioxide emission recovery, the system comprising:
a reformer (412, 512) configured to convert an input into hydrogen and carbon monoxide; and
a methanization reactor (410, 510) coupled to the reformer (412, 512), the methanization reactor (410, 510) configured to:
receive carbon dioxide from one or more off gases of a carbon/carbon (C/C) preform production process;
receive the hydrogen and the carbon monoxide from the reformer (412, 512);
convert the hydrogen, the carbon monoxide, and the carbon dioxide via methanization to produce methane; and
supply the produced methane to at least one of the C/C preform production process or another system for heat generation.

2. The system of claim 1, further comprising:
a condenser coupled to the reformer (412, 512) and the methanization reactor (410, 510), the condenser configured to:
receive the hydrogen, the carbon monoxide, and water from the reformer (412, 512);
condense the water into a liquid form; and
supply the hydrogen and the carbon monoxide to the methanization reactor (410, 510).

3. The system of claim 1 or 2, wherein the input includes a natural gas and steam, or wherein the input includes a hydrocarbon and steam.

4. The system of any preceding claim, further comprising:
a separator coupled to the reformer (412, 512), the separator configured to:
receive one or more off gases from the C/C preform production process;
separate one or more hydrocarbons from the one or more off gases; and
supply the one or more hydrocarbons as the input to the reformer (412, 512), and optionally further comprising:
a steam source coupled to the reformer (412, 512) and configured to provide a steam to the reformer (412, 512); and
a natural gas input coupled to the reformer (412, 512) and configured to provide natural gas to the reformer (412, 512), wherein the input to the reformer (412, 512) includes the steam, the one or more hydrocarbons, and the natural gas are the input to the reformer (412, 512).

5. The system of any preceding claim, further comprising:
a burner/steam generator coupled to the C/C preform production process and the reformer (412, 512), the burner/steam generator configured to:
receive the one or more off gases from the C/C preform production process;
receive an air input;
receive a water input; and
output a steam to the reformer (412, 512), wherein the input to the reformer (412, 512) includes the steam.

6. The system of claim 5, further comprising:
a condenser coupled to the methanization reactor (410, 510) and the burner/steam generator, wherein the methanization reactor (410, 510) produces the methane and steam and wherein the condenser condenses the steam to water which is supplied to the burner/steam generator.

7. A system for carbon dioxide emission recovery, the system comprising:
a carbon/carbon (C/C) preform production component;
a burner/steam generator coupled to the C/C preform production component and configured to receive one or more off gases from the C/C preform production component, receive an air input, and produce steam and carbon dioxide as an output;
a reformer (412, 512) coupled to the burner/steam generator and configured to receive the steam from the burner/steam generator and a hydrocarbon from a hydrocarbon source and output hydrogen and carbon monoxide;
a methanization reactor (410, 510) coupled to the burner/steam generator and the reformer (412, 512), the methanization reactor (410, 510) configured to receive the hydrogen from the reformer (412, 512) and the carbon dioxide from the burner/steam generator and output methane and steam, wherein the methane is supplied back to the C/C preform production component.

8. The system of claim 7, further comprising:
a separator coupled to the C/C preform production component and the reformer (412, 512), the separator configured to receive the one or more off gases and output the hydrogen and hydrocarbons to the reformer (412, 512), wherein the separator is the hydrocarbon source, and optionally further comprising:
a natural gas source coupled to the reformer (412, 512), wherein the natural gas source and the separator are the hydrocarbon source.

9. The system of claim 7 or 8, wherein the hydrocarbon source is a natural gas source.

10. The system of any of claims 7 to 9, further comprising:
a condenser coupled to the reformer (412, 512) and the methanization reactor (410, 510), the condenser configured to:
receive the hydrogen, the carbon monoxide, and water from the reformer (412, 512);
condense the water into a liquid form to be supplied to the burner/steam generator; and
supply the hydrogen and the carbon monoxide to the methanization reactor (410, 510).

11. The system of any of claims 7 to 10, further comprising:
a condenser coupled to the methanization reactor (410, 510) and the burner/steam generator, wherein the methanization reactor (410, 510) produces the methane and steam and wherein the condenser condenses the steam to water which is supplied to the burner/steam generator.

12. The system of any of claims 7 to 10, further comprising:
a separator coupled to the burner/steam generator and the methanization reactor (410, 510) and configured to receive a byproduct from the burner/steam generator, separate the carbon dioxide from the by product, and supply the carbon dioxide to the methanization reactor (410, 510).

13. A method for carbon dioxide emission recovery, the method comprising:
receiving, by a methanization reactor (410, 510), carbon dioxide separated from one or more off gases of a carbon/carbon (C/C) preform production component;
receiving, by the methanization reactor (410, 510), a hydrogen supply from a reformer (412, 512), the hydrogen supply being reformed from a hydrocarbon source;
converting, by the methanization reactor (410, 510), the carbon dioxide and the hydrogen supply via methanization to produce methane; and
supplying, by the methanization reactor (410, 510), the produced methane to the C/C preform production component.

14. The method of claim 13, wherein the hydrocarbon source is a natural gas source, or . wherein the hydrocarbon source is a separator, the separator separating one or more hydrocarbons from the off gases of the C/C preform production component, or wherein the hydrocarbon source includes a natural gas source and a separator, wherein the separator separates the one or more hydrocarbons from the off gases of the C/C preform production component.

15. The method of claim 13 or 14, further comprising:
output, by the methanization reactor (410, 510), steam to a condenser to be converted into liquid water for use by a system for steam generation.
